# EUROPÄISCHE PATENTSCHRIFT

(11) **EP 3 764 935 B1**
(45) Veröffentlichungstag und Bekanntmachung des Hinweises auf die Patenterteilung: **04.05.2022**
(21) Anmeldenummer: 20717113.3
(22) Anmeldetag: 27.03.2020
(51) Int. Cl.: A61B 50/34, B65D 25/28, A61B 50/33, A61B 50/22, A61B 50/00

(54) **BLECHGRIFF UND DECKEL MIT BLECHGRIFF**
SHEET METAL HANDLE, AND COVER WITH SHEET METAL HANDLE
POIGNÉE EN TÔLE ET COUVERCLE AVEC POIGNÉE EN TÔLE

(30) Priorität: 28.03.2019 DE 102019108114
(43) Veröffentlichungstag der Anmeldung: 20.01.2021
(73) Patentinhaber: Aesculap AG, 78532 Tuttlingen (DE)
(72) Erfinder: KLEMM, Svenja, 78609 Tuningen (DE); HENKE, Matthias, 78567 Fridingen (DE)
(74) Vertreter: Winter, Brandl - Partnerschaft mbB
(86) Internationale Anmeldenummer: PCT/EP2020/058811
(87) Internationale Veröffentlichungsnummer: WO 2020/193774

(56) Entgegenhaltungen:
- EP-A2- 3 434 611
- WO-A1-01/78619
- DE-B3-102016 121 723
- DE-U1- 20 105 328
- FR-A1- 2 737 105
- GB-A- 2 103 182
- US-B2- 6 874 634

## Beschreibung

Die vorliegende Erfindung betrifft einen Griff für einen/eines vorzugsweise gitterartig aufgebauten Deckel(s), insbesondere einen Deckel für einen Waschbehälter/ Siebkorb für medizinisches Wasch-/Packgut, einen Deckel mit dem genannten Griff sowie ein Verfahren zum Bilden des genannten Griffs aus einem Blech an dem genannten Deckel.

Als Griff ist hierbei definitionsgemäß eine bügelförmige Schlaufenkonstruktion zu verstehen mit einem steg- oder stabförmigen Handlauf (Griffsteg), an dessen sich gegenüberliegenden Enden jeweils ein Halteschenkel vorzugsweise rechtwinklig zum Handlauf erstrecken, an deren freie Enden zumindest eine Anschlussstruktur, beispielsweise in Form von Scharnierzapfen, Einhängösen oder Schlaufen angeordnet/ausgebildet ist. Die Handlauf-Länge wie auch die Länge der Halteschenkel ist funktionsgemäß so dimensioniert, dass der Handlauf in einem Abschnitt zwischen den (parallel zueinander ausgerichteten) Halteschenkeln in jeder Schwenkposition des Griffs von einer Menschenhand vollumfänglich ergriffen werden kann.

### Hintergrund der Erfindung

Zur Reinigung bzw. zum Waschen medizinischer Instrumente, beispielsweise chirurgische Zangen oder Klemmen, kommen sogenannte "Washtrays" oder Waschkörbe / Siebkörbe / Sterilisiersiebschalen zum Einsatz, die eine Aufnahme bzw. einen Aufnahmebehälter für das Wasch- bzw. Packgut und einen Deckel zum Verschließen dieser Siebkörbe / Waschkörbe haben.

Zur optimalen Umspülung des Waschguts sind die Aufnahmen und Deckel der Waschkörbe gitterartig aufgebaut bzw. bestehen aus einer Gitterstruktur. An den Deckeln der Waschkörbe ist zudem jeweils ein Griff vorgesehen, an dem der Waschkorb bspw. in einen Sterilcontainer hinein versenkt und aus diesem wieder herausgehoben werden kann.

### Stand der Technik

Aus dem Stand der Technik sind Waschkörbe bekannt, die aus einer Gitterstruktur gebildet sind und die eine Aufnahme für medizinisches Waschgut und einen Deckel zum Verschließen der Siebkörbe / Waschkörbe / Sterilisiersiebschalen aufweisen. Die Deckel haben üblicherweise eine im Wesentlichen plane Oberseite, an der ein Tragegriff angeordnet ist.

EP 3 434 611 A2 offenbart ein Stückhaltesystem für den industriellen Einsatz, das in den industriellen Betriebsphasen eingesetzt werden soll, die sich auf den Transport, die Lagerung und die Reinigung von ordnungsgemäß angeordneten Teilen beziehen, insbesondere in den Industriebereichen Metallbearbeitung, Mechatronik, Präzisionsmechanik, Mikroelektronik, Robotik, in der medizinischen und pharmazeutischen Industrie. Das Stückhaltesystem sieht die Verwendung eines aus einem Boden und Seitenwänden bestehenden Behälters als Mittel zum Aufnehmen und / oder Abstützen der Teile vor. Dabei sind auf dem Boden mindestens eine der Seitenwände Gitter angeordnet, bei denen geformte Elemente einführbar sind, die von den Oberflächen vorstehen, um die in dem Behälter abgelagerten Teile zurückzuhalten.

WO 01/78619 A1 betrifft eine Vorrichtung zur Aufbewahrung von chirurgischen Instrumenten und/oder Implantaten mit mindestens einem schalenförmigen Instrumententablar und einem auf dessen Oberseite verschliessbaren Deckel.

DE 10 2016 121723 B3 betrifft einen Sterilgutkorb mit einer zwei lange Seiten und zwei kurze Seiten aufweisenden rechteckigen Grundfläche und mit zwei an einem oberen Rand angeordneten Klappbügeln, welche in einer ersten Klappposition ein Ineinanderstapeln und in einer zweiten Klappposition mit jeweils einer horizontalen Halteleiste ein Übereinanderstapeln ermöglichen. Erfindungsgemäß sind die Klappbügel an den langen Seiten des Sterilgutkorbes angeordnet.

GB 2 103 182 A offenbart einen Behälter mit Beinen, die aus einer Griff- oder Lagerposition in eine bestimmte Position drehbar und aus dieser Position durch Schwerkraftwirkung in einen Stützzustand verlagerbar sind. Stangen an den Beinen greifen unter Haken ein, um die Beine in Position zu halten. Die Stangen und Haken können in einer modifizierten Konstruktion ausgetauscht werden. Vorsprünge ermöglichen das Stapeln der Behälter.

Unabhängig von der Einzel- oder Mehrfachnutzung eines Containers muss bei der Dimensionierung des Containerinnenraums und/oder des Waschkorbs bzw. der Waschkörbe der Tragegriff am Deckel des Waschkorbs mitberücksichtigt werden, da dieser aufgrund seiner eigenen Höhe zusätzlichen Raum im Containerinneren notwendig macht.

Da zusätzlicher Raum mit einem höherem Materialbedarf beim Bau des Containers verbunden ist (Containerhöhe), gilt es, den Containerinnenraum möglichst vollständig auszunutzen und insbesondere ungenutzten Raum zwischen der Deckeloberseite des Waschkorbdeckels und der Deckelunterseite des Containerdeckels bzw. zwischen der Deckeloberseite eines unteren Waschkorbs und dem Boden eines darüber gestapelten Waschkorbs zu reduzieren.

Dieses Problem ist aus dem Stand der Technik bereits bekannt und wird begrenzt, indem anstelle von feststehenden Griffen an der Deckeloberseite Fallgriffe angebracht werden. Diese Fallgriffe gemäß der eingangs gegebenen Definition werden gewöhnlicher Weise aus einem Rundmaterial, beispielsweise einer Metallstange runden Querschnitts, gefertigt. Das Rundmaterial wird dann zu einem Griff in Gestalt eines gebogenen Rundstabs geformt, indem die beiden Enden des Rundmaterials abgewinkelt bzw. umgebogen werden und durch Biegen des zwischen den beiden Enden befindlichen Rundmaterials koaxial (parallel) zueinander ausgerichtet werden.

Der in Gestalt eines gebogenen Rundstabs gebildete Griff wird dann mit dem Deckel des Waschkorbs verbunden, indem aus dem Deckelblech, durch Aufrollen eine Griffaufhängung gebildet wird, welche die abgewinkelten Enden des Griffs umgreift. Innerhalb der Griffaufhängung kann der Griff dann scharnierartig gedreht und relativ zur Deckeloberseite verschwenkt werden, sodass der Griff beim Transport des Deckels bzw. des Waschkorbs eine aufrechte Position einnehmen kann und beim Schließen des Containerdeckels über dem im Containerinneren platzierten Waschkorb und/oder beim Stapeln mehrerer Waschkörbe übereinander flach auf den Deckel geschwenkt oder geklappt werden kann.

Dennoch baut das zu einer Aufhängung aufgerollte Deckelblech, welches die abgewinkelten Enden des gebogenen Rundstabs umfangsseitig umgreifen muss, insgesamt eine Höhe von mehr als 5mm auf die Deckeloberseite auf, sodass beim Schließen des Containerdeckels über dem Waschkorb und/oder beim Stapeln der Waschkörbe übereinander zwischen der Deckeloberseite des Waschkorbs und dem Containerdeckel bzw. Boden des darüber angeordneten Waschkorbs ein Spalt von mehr als 5mm verbleibt. Das Problem der mangelhaften Raumausnutzung im Container wird folglich nur unzureichend reduziert.

### Kurze Beschreibung der Erfindung

Die Aufgabe der vorliegenden Erfindung ist es somit, die Nachteile des Stands der Technik zu überwinden oder zumindest zu mildern und insbesondere einen (Hand-)Griff gemäß eingangs gegebener Definition bzw. einen Deckel mit (Hand-)Griff für einen medizinischen Siebkorb zu schaffen, der es ermöglicht, den Innenraum eines Containers beim Befüllen mit mindestens einem Siebkorb möglichst vollständig auszunutzen und/oder beim Stapeln mehrerer Siebkörbe übereinander das Spaltmaß zwischen der Deckeloberseite eines unteren Siebkorbs und dem Boden eines darüber angeordneten Siebkorbs zu reduzieren. Insbesondere soll dabei der nutzbare Innenraum des Siebkorbs nicht oder nur gering beeinträchtigt werden. Zudem soll der Griff in bevorzugter Weise einfach herzustellen und am Deckel zu montieren sein. Weiter sollen bevorzugt insbesondere Spülschatten vermieden und eine Möglichkeit zum automatischen Einnehmen einer raumsparenden Position des Griffs geschaffen werden.

Die Aufgabe wird gelöst durch einen (Hand-)Griff gemäß der eingangs gegebenen Definition mit den Merkmalen des Anspruchs 1, einen Deckel mit den Merkmalen des Anspruchs 8 sowie ein Verfahren mit den Merkmalen des Anspruchs 14. Vorteilhafte Weiterbildungen der Erfindung sind Gegenstände der beigefügten Unteransprüche.

Ein Grundgedanke der Erfindung besteht darin, einen möglichst flachen (Hand-) Griff für einen/eines Deckel(s) für einen/eines Siebkorb(s)/Waschkorb(s) zu schaffen, dessen Befestigungsstruktur am Deckel keinen oder nur einen geringen Höhenaufbau bedingt. Am Deckel montiert soll der Griff eine Position einnehmen können, in der der montierte Griff an der Deckeloberseite möglichst wenig Höhe aufbaut, insbesondere ohne bzw. nur in geringem Maß den nutzbaren Innenraum im Waschkorb zu reduzieren, indem der Griff teilweise in den Deckel hinein versenkt wird. Der Griff soll zudem durch Umformen eines (zugeschnittenen) Blechs bzw. Blechrohlings hergestellt und am Deckel montiert werden können.

Genauer ausgedrückt besteht der erfindungsgemäße (Deckelhand-)Griff für einen/eines medizinischen Siebkorb(s) aus einem zu einem Grundprofil / Grundkörper ausgearbeiteten (ausgestanzten / ausgeschnittenen) dünnwandigen Blech (Metallblech) aufweisend oder bestehend aus einem (oberen) Griffsteg mit einem sich an diesen anschließenden Halteabschnitt, der vorzugsweise zwei Haltearme / Halteschenkel aufweist, die sich beidseits des Griffstegs (rechtwinklig) an diesen anschließen und deren freie Arm-Enden zumindest teilweise zu Lagerösen / Fixierungslaschen umgebogen sind, bzw. an deren freie Arm-Enden Lagerösen / Fixierungslaschen angeordnet sind, sowie ggf. einem (mittleren oder unteren) Aussteifungssteg im Bereich zwischen dem Griffsteg und den Lagerösen oder unmittelbar angrenzend an die Lagerösen, welcher die beiden Haltearme / Halteschenkel aussteifend (stoffeinstückig) verbindet.

Dies Konstruktion erlaubt es, den Griffsteg, die Haltearme und die Lagerösen/Fixierungslaschen aus dem gleichen (dünnwandigen) Blechmaterial vorzugsweise stoffeinstückig herzustellen, wobei der Aussteifungssteg (erstreckt sich im Wesentlichen parallel zum Griffsteg), der ebenfalls aus dem gleichen (dünnwandigen) Blechmaterial vorzugsweise stoffenstückig mit den Haltearmen hergestellt ist, die nötige Biege-/Verwindungssteifigkeit des (Hand-)Griffs bereitstellt.

Vorzugsweise ist an einem Innenkantenabschnitt des Griffstegs eine (Griff-)Mulde / Grifflasche umgebogen, wodurch ein Einschneiden einer infolge des dünnwandigen Blechmaterials entstehenden scharfen Griffkante in eine Menschenhand beim vollumfänglichen Ergreifen des Griffstegs vermieden wird.

Weiter vorzugsweise ist die Biegungsrichtung sämtlicher umgebogener Abschnitte des (Hand-)Griffs die gleiche, sodass alle Abbiegungen in die gleiche Richtung vorzugsweise in einem einzigen Biegeschritt (ohne Umlegen des Blechrohlings) erfolgen kann.

In anderen Worten ist ein (Hand-)Griff für einen vorzugsweise gitterartig aufgebauten Behälterdeckel vorgesehen, der in seiner Grundform aus einem entsprechend zugeschnittenen Blech besteht bzw. gefertigt ist, beispielsweise in Form eines (im Wesentlichen) rechteckigen geschlossenen Rings. Dabei hat der Griff einen im Wesentlichen planen (ungebogenen) Grundkörper (aus dem dünnwandigen Blechmaterial), der an einer bzw. einer ersten Außenkante mindestens einen laschenförmigen Vorsprung (stoffeinstückig) hat/ausbildet, der zu einer Fixierungslasche/Öse umgebogen ist, welche dazu vorgesehen und angepasst ist, den (Hand-)Griff schwenkbar an einem Deckel zu sichern, vorzugsweise durch (zumindest teilweises) Umgreifen/Umschließen eines dorn- oder stabförmigen Teils des Deckels. Somit bildet die zumindest eine umgebogene Fixierungslasche durch Umgreifen eines Teils des Deckels über ihre Breite ein Schwenkscharnier aus, an dem der Griff relativ zum Deckel ausgelenkt werden kann.

Insbesondere ist die mindestens eine Fixierungslasche um eine Befestigungsstrebe am Deckel herum gebogen und bildet (im montierten Zustand) zwischen sich und dem Halteabschnitt/Halteschenkel einen (engen) Spalt oder das freie Ende der jeweiligen gebogenen Lasche liegt unmittelbar an dem Halteabschnitt/Halteschenkel stoßartig an.

Konkret ist ein (Hand-)Griff vorzugsweise für einen/eines Deckel(s) für einen/eines medizinischen Siebkorb(s) vorgesehen, mit einem Grundkörper (aus einem dünnwandigen Blechmaterial), der einen Griffsteg und einen sich an den Griffsteg (stoffeinstückig) anschließenden bzw. mit diesem verbundenen Halteabschnitt (ebenfalls aus dem dünnwandigen Blechmaterial) aufweist oder aus diesen besteht, und mindestens einer Ösen-förmigen oder gebogenen Fixierungslasche (ebenfalls aus dem dünnwandigen Blechmaterial), die an einem dem Griffsteg entgegengesetzten Ende des Halteabschnitts zur Schwenkmontage des Griffs (stoffeinstückig) angeordnet ist. Hierfür ist der Grundkörper des Griffs aus einem (im Wesentlichen) planen (ungebogenen und/oder zugeschnittenen) Metallblech gebildet, welches an dem Griffsteg entgegensetzten Ende des Halteabschnitts zu zumindest einem, diesen verlängernden, laschenartigen Vorsprung ausgearbeitet ist, welcher zur Ausbildung der Fixierungslasche umgebogen ist.

Insbesondere ist die Fixierungslasche in Bezug auf den Halteabschnitt bzw. Grundkörper so umgebogen, dass ihr freies Ende zu einer Unterseite des Halteabschnitts bzw. Grundkörpers hin ausgerichtet ist. Vorzugsweise ist die Oberseite des Griffs eine plane Auflagerfläche, die (im Wesentlichen) planparallel zur Unterseite des Grundkörpers verläuft, und die Fixierungslasche erstreckt sich in einer Richtung senkrecht zur Auflagerfläche nicht über die Auflagerfläche hinaus.

Zusammengefasst handelt es sich bei dem Griff also um ein zunächst ungebogenes (dünnwandiges) Blechteil, das in eine vorgesehene, (im Wesentlichen rechteckige) ringförmige Grundform bestehend aus dem Griffsteg, dem zweischenkligen Halteabschnitt und der mindestens einen am Halteabschnitt vorspringenden Lasche geschnitten ist, wobei die vorspringende Lasche in Bezug auf den Halteabschnitt (zu einer Öse) umgebogen ist. Der übrige Teil der Grundform (Griffsteg und Halteabschnitt) bleiben zunächst ungebogen. Dadurch entsteht der beschriebene, für ein Metallblech typische plane, ungebogene Grundkörper, der zur Schwenkmontage des Griffs an seinem einen Ende die umgebogene Fixierungslasche aufweist.

Vorteile werden mindestens dahingehend erzielt, dass der (Hand-)Griff in einer Ruheposition bzw. Anlageposition am Deckel, in der der Griff mit der Unterseite seines Grundkörpers auf dem Deckel anliegt, nur eine Höhe in Form seiner eigenen Blechstärke auf den Deckel aufbaut (insbesondere weil die Fixierungslasche in Richtung zur Unterseite des Griffs umgebogen ist und somit in der vorstehend definierten Ruheposition in den Deckel hineinragt). Weiter verursacht der am Deckel anliegende plane Grundkörper keinen bzw. vernachlässigbaren Spülschatten, was eine bessere Reinigung ermöglicht.

Bei dem Blech handelt es sich insbesondere um ein Blech von (im Wesentlichen) gleichbleibender Wandstärke, vorzugsweise von 1,25 mm. Auf diese Weise kann eine ausreichend hohe (Verwindungs-)Stabilität bei möglichst geringer Blechstärke und folglich möglichst geringem Höhenaufbau erreicht werden.

Vorzugsweise weist der Halteabschnitt zwei Halteschenkel auf oder besteht aus diesen, die an den freien Enden des Griffstegs angeordnet sind und an deren freien Enden / Endabschnitten jeweils eine Fixierungslasche angeordnet/ausgebildet ist. Insbesondere schließt sich an den freien Enden des Griffstegs jeweils ein Halteschenkel vorzugsweise im Wesentlichen senkrecht an diesen an.

Dabei können die Halteschenkel insbesondere spiegelbildlich zueinander geformt und/oder I-förmig oder L-förmig sein.

Auf diese Art wird die Belastung / Zugkraft, die beim Tragen des mit dem Deckel verbundenen Waschkorbs auf den Griff aufgebracht wird, vorteilhafterweise gleichmäßig über den Griff verteilt.

Insbesondere weist der Halteabschnitt weiter einen Aussteifungssteg auf, der die zwei Halteschenkel zwischen Griffsteg und Fixierungslasche, vorzugsweise im Bereich der freien Enden der Halteschenkel, stoffeinstückig verbindet. Besonders bevorzugt grenzt der Aussteifungssteg unmittelbar an die Fixierungslaschen an.

Auf diese Weise wird die Stabilität und Festigkeit des Griffs weiter verbessert. Klarstellungshalber sei an dieser Stelle angemerkt, dass auch der Aussteifungssteg Teil (stoffeinstückig) des planen Metallblechs und ungebogen ist.

Besonders bevorzugt ist an einem Innenkantenabschnitt des Griffstegs zumindest eine rinnen- oder muldenförmige Greiflasche ausgebildet. Dazu kann an dem Innenkantenabschnitt ein Blechband, das den Griffsteg in Richtung hin zum zumindest einen laschenartigen Vorsprung verbreitert, ausgeformt und umgebogen sein.

Auf diese Weise wird ein einfach zu konstruierender, besonders komfortabler Greifabschnitt gebildet.

Unter der Innenkante ist die Kante des Griffstegs zu verstehen, die der mindestens einen Fixierungslasche zugewandt ist. Auch wird an dieser Stelle klarstellungshalber festgehalten, dass die Greiflasche Teil des Metallblechkörpers ist, aus dem der Griff gefertigt ist, und die Greiflasche somit stoffeinstückig mit dem Griffsteg, in Bezug auf den sie umgebogen ist, verbunden ist. So wie die mindestens eine Fixierungslasche ragt auch die umgebogene Greiflasche in der Richtung senkrecht zur Oberseite des Griffs nicht über diese hinaus.

Ein weiterer ggf. unabhängig beanspruchbarer Aspekt der vorliegenden Erfindung betrifft die Ausgestaltung der Fixierungslasche bzw. Öse.

Demzufolge hat der erfindungsgemäße Griff einen Grundkörper aus einem (dünnwandigen) Metallblech, der sich aus einem Griffsteg und zwei Halteschenkeln zusammensetzt, die sich an den Enden des Griffstegs in einem im Wesentlichen rechten Winkel zu diesem erstrecken und an ihren freien Enden zu den Fixierungslaschen/Ösen umgebogen sind. Optional ist ein Aussteifungssteg zwischen den Halteschenkeln (nahe den Fixierungslaschen) stoffeinstückig mit den Halteschenkeln ausgebildet, der sich im Wesentlichen parallel zum Griffsteg erstreckt und die beiden Halteschenkel im Bereich ihrer freien Enden miteinander verbindet.

Die aus dem planen Metallblech sowie in Verlängerung der Halteschenkel gebogenen Fixierungslaschen weisen dabei folgende Längsabschnitte in der angegebenen Reihenfolge ausgehend vom jeweiligen Halteschenkel auf:
- ein erster Krümmungsabschnitt, der die Fixierungslasche ausgehend vom Halteschenkel um einen ersten stumpfen Winkel vorzugsweise zwischen 90° und 110° zum Halteschenkel abkrümmt,
- einen ersten geraden Abschnitt von einer ersten Länge, der sich an den ersten Krümmungsabschnitt anschließt,
- einen zweiten Krümmungsabschnitt, der den ersten geraden Abschnitt um einen zweiten stumpfen Winkel in die gleiche Abkrümmrichtung wie der erste Krümmungsabschnitt abkrümmt, wobei der zweite stumpfe Winkel größer ist als der erste stumpfe Winkel, vorzugsweise zwischen 120 und 150°,
- einen zweiten geraden Abschnitt, der sich an den zweiten Krümmungsabschnitt anschließt und eine zweite Länge hat, die größer ist als die erste Länge,
- einen dritten Krümmungsabschnitt, der den zweiten geraden Abschnitt um einen dritten stumpfen Winkel in die gleiche Abkrümmrichtung wie der zweite Krümmungsabschnitt abkrümmt, wobei der dritte stumpfe Winkel im Wesentlichen dem ersten stumpfen Winkel entspricht, der Krümmungsradius des dritten Krümmungsabschnitts jedoch größer ist als der Krümmungsradius des ersten Krümmungsabschnitts und
- einen dritten geraden Abschnitt, der sich an den dritten Krümmungsabschnitt anschließt und eine dritte Länge hat, die im Wesentlichen der ersten Länge entspricht.

Diese Formgebung ermöglicht es, insbesondere im Fall eines Schwenkzapfens ebenfalls aus einem planen dünnwandigen Metallblech die Breite (nicht Blechwandstärke) des Schwenkzapfens derart auf die inneren Abmessungen der so geformten Öse anzustimmen, dass ein Drehen der Öse um den (laschenförmigen) Schwenkzapfen bis zu einem bestimmten Schwenkwinkel möglich ist, bei dessen Erreichen der Schwenkzapfen in der Öse verkeilt und so ein Weiterschwenken verhindert. Je nach Drehausrichtung des (laschenförmigen) Schwenkzapfens entspricht der Verkeilungswinkel dem maximalen Aufstell-/Aufschwenkwinkel des (Hand-)Griffs bezüglich des Deckels, an welchem der (Hand)-Griff anscharniert ist.

Erfindungsgemäß ist weiter ein Deckel für einen/eines Behälter(s), insbesondere für einen/eines medizinischen Siebkorb(s), mit einem wie vorstehend beschriebenen Griff vorgesehen. Der Deckel weist weiter zumindest eine Befestigungsstrebe oder zumindest einen Scharnierstift auf, die/der von der mindestens einen Fixierungslasche umfangsseitig (entlang des Umfangs der Befestigungsstrebe abschnittsweise oder vollständig) umgriffen/umschlossen ist und an der/dem der Griff schwenkbar befestigt ist.

Bei einem gitterartig aufgebauten Deckel kann die Befestigungsstrebe Teil der Deckel-Gitterstruktur sein. Bei unvollständigem Umschließen der Umfangsseite der Befestigungsstrebe durch den Griff ist die Öffnung der Fixierungslasche bzw. der Spalt zwischen dem freien Ende der Fixierungslasche und dem Halteabschnitt (bzw. Grundkörper) in montiertem Zustand des Griffs so dimensioniert, dass die Befestigungsstrebe nicht durch die Öffnung herausgenommen werden kann.

In dieser Deckel-Griff-Konstruktion kann der Griff vorteilhafterweise flach am Deckel anliegen und baut nicht mehr Höhe als seine eigene Blechstärke, insbesondere die Blechstärke seines planen Grundkörpers, auf den Deckel auf.

Insbesondere befindet sich die mindestens eine Fixierungslasche in einer Ruheposition des Griffs, in der dieser mit seinem Grundkörper am Deckel anliegt, auf der dem Deckel zugewandten Griffseite, welche als Unterseite des Griffs zu verstehen ist.

Die Höhe der gebogenen Fixierungslasche baut vorteilhafterweise somit nicht auf den Deckel auf.

Insbesondere weist der Deckel weiter eine Ausnehmung oder Mulde auf, die so positioniert und dazu vorgesehen und angepasst ist, dass die Greiflasche in der Ruheposition des Griffs (vollständig) in die Ausnehmung oder Mulde hinein versenkt ist.

Weiter bevorzugt ist die Deckelseite, die in der Ruheposition des Griffs dem Griff zugewandt ist, eine Deckeloberseite und ragt die Befestigungsstrebe nicht über die (übrige) Deckeloberseite hinaus. Insbesondere ist die Befestigungsstrebe bündig mit der (übrigen) Deckeloberseite.

Dadurch wird ermöglicht, dass der Grundkörper des Griffs (nahezu) lückenlos / spaltfrei auf dem Deckel aufliegt.

Außerdem bevorzugt ist die Auslenkung / Verschwenkung des Griffs relativ zum Deckel auf einen maximalen Auslenkungs- / Schwenkwinkel von 80° bis 89°, besonders bevorzugt 85°, begrenzt. Dies kann beispielsweise dadurch realisiert sein, dass bei einem Auslenkungs- / Schwenkwinkel von 80° bis 89°, insbesondere 85° eine Außenkante des Halteabschnitts mit dem Deckel (Deckeloberseite) in Anlage tritt, wodurch der Griff in diesem Winkel am Deckel anschlägt und die Auslenkung auf ein Maximum begrenzt wird.

Konkret ist bevorzugt die Relativlage zwischen der dem Deckel zugewandten Unterkante des Halteabschnitts, insbesondere des Aussteifungsstegs, und der mindestens einen Fixierungs-/Schwenklasche derart bestimmt, dass mit Erreichen des maximalen Auslenkungswinkels die Unterkante auf der Deckeloberseite anschlägt.

Somit wird ermöglicht, dass der Griff nicht bis zu einer senkrechten Stellung relativ zur Deckeloberseite ausgelenkt werden kann und der Griff folglich wieder in seine Ruheposition zurückfällt, wenn eine äußere Krafteinwirkung, die den Griff auslenkt, wegfällt. Es wird folglich verhindert, dass der Griff, wenn er zum Tragen des Waschkorbs nicht mehr benötigt wird, beim Aufstapeln eines weiteren Waschkorbs oder beim Schließen des Containers mit einem Containerdeckel stört.

Weiter betrifft die Erfindung ein Verfahren zum Bilden eines wie vorstehend beschriebenen Griffs an einem wie vorstehend beschriebenen Deckel aus einem (planen, zugeschnittenen) Blech bzw. Blechrohling, das bzw. der einen Grundkörper bestehend aus einem Griffsteg und zumindest einem sich an diesen anschließenden Halteabschnitt und zumindest einen, den Halteabschnitt verlängernden, laschenartigen Vorsprung mit einem vom Halteabschnitt abgewandten freien Ende aufweist, mit den Schritten:
Umformen des zumindest einen laschenartigen Vorsprungs zu einer gebogenen Fixierungslasche derart, dass diese eine offene, gekrümmte Form einnimmt;
Einbringen der Befestigungsstrebe in die offene, gekrümmte Fixierungslasche, sodass diese die Befestigungsstrebe offen umgreift; und
Umformen der offenen Fixierungslasche derart, dass das freie Ende der Fixierungslasche näher an den Grundkörper angeordnet wird und/oder das freie Ende am Grundkörper zum Liegen kommt und die Befestigungsstrebe von der Fixierungslasche (nahezu vollständig) umschlossen und schwenkbar gesichert wird.

Vorzugsweise ist der Blechrohling an einer Innenkante des Griffstegs zu zumindest einem, diesen in Richtung hin zum laschenartigen Vorsprung verbreiternden Blechband ausgeformt und weist das Verfahren weiter den folgenden Schritt Umformen des Blechbands derart, dass dieses eine umgebogene Greiflasche bildet, auf.

Auf diese Weise kann der vorstehend beschriebene Griff aus einem einzigen Bauteil (Metallblech) gefertigt und der vorstehend beschriebene Deckel mit Griff durch einfache Verfahrensschritte realisiert werden.

### Kurzbeschreibung der Figuren

Die Erfindung wird nachstehend anhand von bevorzugten Ausführungsbeispielen unter Bezugnahme auf die beigefügte Zeichnung näher beschrieben. Es zeigen:
Fig. 1 eine perspektivische Ansicht eines Griffs in einer erfindungsgemäßen Ausführungsform;
Fig. 2 eine Draufsicht auf den Griff in der erfindungsgemäßen Ausführungsform;
Fig. 3 eine Seitenansicht des in Fig. 2 gezeigten Griffs in der erfindungsgemäßen Ausführungsform;
Fig. 4 eine vergrößerte Ansicht des Bereichs IV der Fig. 3;
Fig. 5 eine perspektivische Ansicht eines Deckels in einer erfindungsgemäßen Ausführungsform in einer ausgelenkten Position des Griffs;
Fig. 6 eine Draufsicht auf den Deckel in der in Fig. 5 gezeigten ausgelenkten Position des Griffs;
Fig. 7a eine Schnittansicht des Deckels und Griffs in der erfindungsgemäßen Ausführungsform entlang der Linie Vlla der Fig. 6;
Fig. 7b eine vergrößerte Darstellung des Bereichs Vllb der Fig. 7a;
Fig. 8a eine Schnittansicht des Deckels und Griffs in der erfindungsgemäßen Ausführungsform entlang der Linie VIIIa der Fig. 6;
Fig. 8b eine vergrößerte Darstellung des Bereichs Vlllb der Fig. 8a;
Fig. 9 eine perspektivische Draufsicht des Deckels in der erfindungsgemäßen Ausführungsform in der Ruheposition des Griffs;
Fig. 10 eine Seitenansicht des in Fig. 9 gezeigten Deckels in der erfindungsgemäßen Ausführungsform;
Fig. 11 eine Schnittansicht des Griffs in der erfindungsgemäßen Ausführungsform im Bereich einer Fixierungslasche in der Ruheposition des Griffs;
Fig. 12 eine Draufsicht auf ein Blech, aus dem der Griff der erfindungsgemäßen Ausführungsform gefertigt wird.

Die Figuren sind lediglich schematischer Natur und dienen ausschließlich dem Verständnis der Erfindung. Die gleichen Elemente sind mit denselben Bezugszeichen bezeichnet.

### Detaillierte Beschreibung bevorzugter Ausführungsbeispiele

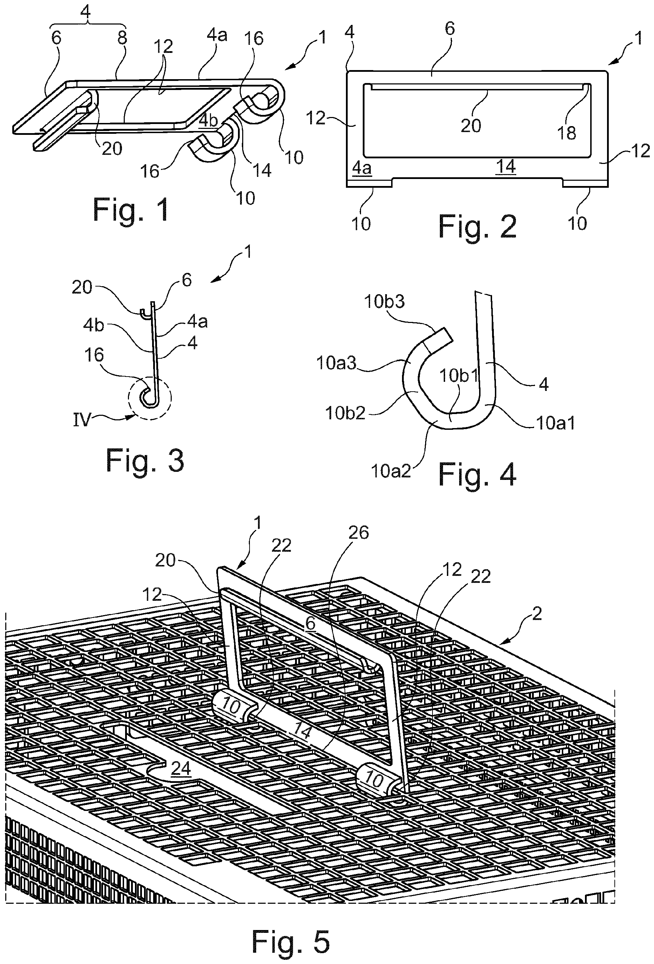

In Fig. 1 ist eine perspektivische Ansicht eines Griffs 1 in einer erfindungsgemäßen Ausführungsform gezeigt. Der Griff 1 ist für einen Deckel 2 (siehe Fig. 5) eines medizinischen Siebkorbs vorgesehen und besteht aus einem Grundkörper 4, der einen Griffsteg 6 und einen sich an den Griffsteg anschließenden Halteabschnitt 8 aufweist, und zwei Ösen-förmigen oder gebogenen Fixierungslaschen 10, die an einem dem Griffsteg 6 entgegengesetzten Ende des Halteabschnitts 8 zur Schwenkmontage des Griffs 1 angeordnet sind. Der Grundkörper 4 des Griffs 1 besteht aus einem planen Metallblech, welches an dem dem Griffsteg 6 entgegengesetzten Ende des Halteabschnitts 8 zu zwei den Haltenabschnitt 8 verlängernden, laschenartigen Vorsprüngen ausgearbeitet ist, welche zur Ausbildung der Fixierungslaschen 10 umgebogen sind. Der Halteabschnitt 8 weist zwei Halteschenkel 12 auf, an deren freien Enden jeweils eine Fixierungslasche 10 angeordnet ist. Die Halteschenkel 12 schließen sich jeweils an ein freies Ende des Griffstegs 6 rechtwinklig an. Zwischen den Halteschenkeln 12 befindet sich ein Aussteifungssteg 14, der die beiden Halteschenkel 12 miteinander verbindet und unmittelbar an die Fixierungslaschen 10 angrenzt.

Der im Wesentlichen rechteckig geformte, plane Grundkörper 4 weist eine plane Oberseite 4a und eine planparallel zur Oberseite 4a verlaufende Unterseite 4b auf. Die umgebogenen Fixierungslaschen 10 erstrecken sich zunächst im Wesentlichen senkrecht zur Unterseite 4b und nehmen in ihrem weiteren Verlauf eine in Bezug auf die Unterseite 4b konvex gekrümmte Form ein, sodass ein freies Ende 16 der Fixierungslaschen 10 der Unterseite 4b zugewandt ist.

An einer Innenkante 18 (zu sehen in Fig. 2) des Griffstegs 6 ist eine umgebogene Greiflasche 20 vorgesehen, die sich ebenfalls zunächst im Wesentlichen senkrecht zur Unterseite 4b erstreckt und sich in ihrem weiteren Verlauf im Wesentlichen parallel zur Unterseite 4b ausrichtet, sodass die Greiflasche 20 eine Rinne oder Mulde bildet. Die Greiflasche 20 und der Griffsteg 6 bildet zusammen den Greifabschnitt, an dem ein Benutzer den Griff (um)fasst, um den Deckel bzw. den mit dem Deckel verschlossenen Behälter zu tragen.

Die gezeigte Gestalt des Griffs 1 entspricht einem Halbfertigprodukt des Griffs 1, bevor dieser mit einem Deckel 2 verbunden wird. Bei dem Halbfertigprodukt weisen die freien Enden 16 der Fixierungslaschen 10 einen deutlichen Abstand zur Unterseite 4b auf.

Fig. 2 ist eine Draufsicht auf den Griff 1 in der erfindungsgemäßen Ausführungsform. Zu sehen ist der Grundkörper 4 von seiner Oberseite 4a. Die beiden Fixierungslaschen 10 sind so an den Halteschenkeln 12 angeordnet, dass ihre Außenkanten bündig mit den Außenkanten der Halteschenkel 12 verlaufen. Zu sehen ist weiter, dass der Aussteifungssteg 14 unmittelbar an die Fixierungslaschen 10 angrenzt. Die Greiflasche 20 erstreckt sich entlang eines Abschnitts der Innenkante 18 und ist mittig an dieser angeordnet.

Fig. 3 zeigt eine Seitenansicht des in Fig. 2 gezeigten Griffs in der erfindungsgemäßen Ausführungsform. Erkennbar ist insbesondere der planparallele Verlauf der Oberseite 4a und der Unterseite 4b des Grundkörpers 4, der Abstand des freien Endes 16 der Fixierungslasche 10 zur Unterseite 4b und die Greiflasche 20, die zusammen mit dem Griffsteg 6 den Greifabschnitt des Griffs 1 bildet.

In Fig. 4 ist eine vergrößerte Ansicht des Bereichs IV der Fig. 3 gezeigt. Wie aus Fig. 4 zu erkennen ist, weist der Verlauf der umgebogenen Fixierungslasche 10 sowohl gekrümmte Abschnitte 10a1, 10a2, 10a3, als auch lineare Abschnitte 10b1, 10b2, 10b3auf. Dabei sind die gekrümmten Abschnitte 10a1-3 und die linearen Abschnitte 10b1-3 abwechselnd angeordnet. Die Krümmungsradien der gekrümmten Abschnitte 10a1-3 können gleich und unterschiedlich sein. Insbesondere kann der Krümmungsradius des gekrümmten Abschnitts 10a1, der sich an den Grundkörpers 4 anschließt, kleiner als die übrigen zwei gekrümmten Abschnitte 10a2,3 sein, welche denselben Krümmungsradius haben.

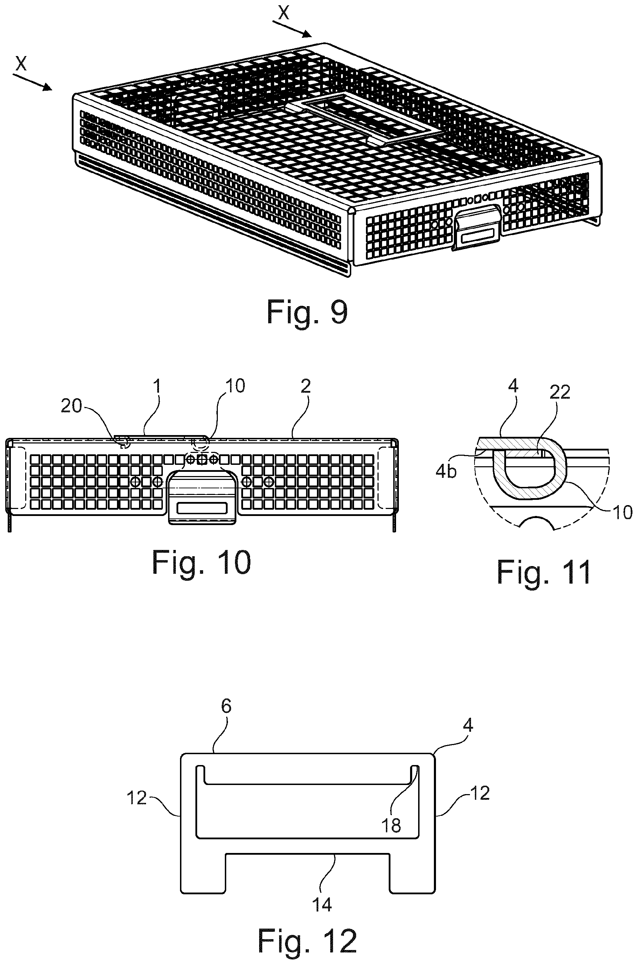

In Fig. 5 ist eine perspektivische Ansicht eines Deckels 2 mit einem Griff 1 in einer erfindungsgemäßen Ausführungsform gezeigt. Der Griff 1 befindet sich in einer ausgelenkten Position. Zu sehen ist der Griff 1 von seiner Unterseite 4b. Der Deckel 2 ist in Form einer Gitterstruktur gebildet. Zu sehen ist, dass der Griff 1 mit seinem Fixierungslaschen 10 am Deckel 2 befestigt / montiert ist, indem die Fixierungslaschen 10 jeweils eine in die Gitterstruktur des Deckels 2 integrierte Befestigungsstrebe 22 umgreifen / umschließen. Die freien Enden 16 der Fixierungslaschen 10 liegen an der Unterseite 4b an. Auf diese Weise bilden die Fixierungslaschen 10 jeweils ein Scharnier und die Befestigungsstreben 22 jeweils einen Scharnierstift, wodurch der Griff 1 schwenkbar am Deckel 2 gehalten ist. In der dargestellten Position befindet sich der Griff 1 in einer maximal ausgelenkten Stellung relativ zur Deckeloberseite. In der abgebildeten maximal ausgelenkten Stellung des Griffs 1 tritt eine Außenkante 26 des Aussteifungsstegs 14, genauer gesagt die Außenkante des Aussteifungsstegs 14 an der Unterseite 4b, in Anlage mit dem Deckel 2 / der Deckeloberseite und begrenzt dadurch den Schwenk- oder Auslenkungswinkel. Der maximale Schwenk- oder Auslenkungswinkel beträgt 85°. Zu sehen ist weiter eine Ausnehmung 24 im Deckel 2. Die Ausnehmung 24 ist so am Deckel 2 positioniert und dimensioniert, dass die Greiflasche 20 beim Zurückschwenken des Griffs 1 in seine Ruheposition in die Ausnehmung 24 eintauchen und in dieser aufgenommen werden kann (siehe Figuren 9 und 10).

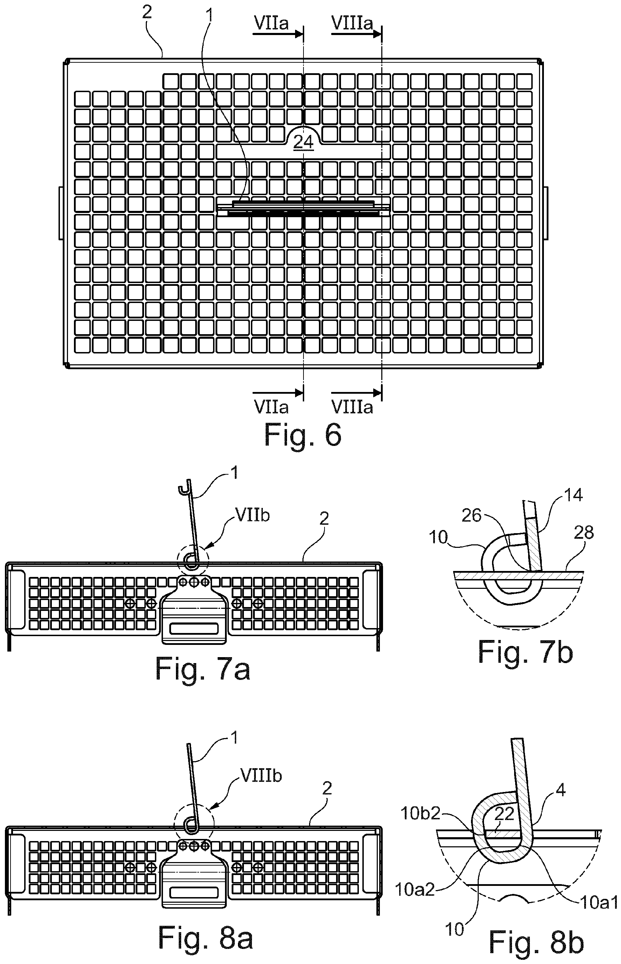

In Fig. 6 ist eine Draufsicht auf den Deckel 2 in der in Fig. 5 gezeigten ausgelenkten Position des Griffs 1 gezeigt.

Fig. 7a zeigt eine Schnittansicht des Deckels 2 mit montiertem Griff 1 entlang der Linie Vlla der Fig. 6. Der Griff 1 ist relativ zum Deckel 2 maximal ausgelenkt.

In Fig. 7b ist eine vergrößerte Darstellung des Bereichs VIIb der Fig. 7a gezeigt. Zu sehen ist, dass die Außenkante 26 des Aussteifungsstegs 14 den Schwenkwinkel des Griffs 1 relativ zum Deckel 2 begrenzt, indem sie mit einer Gitterstrebe 28 des Deckels 2 in Anlage tritt. Die gezeigte Gitterstrebe 28 bildet folglich einen Anschlag zur Begrenzung des Schwenkwinkels. Da der maximale Schwenkwinkel 85°beträgt, fällt der Griff 1 nach Wegfall der äußeren Kraft, die den Griff 1 aus seiner Ruheposition auslenkt, schwerkraftbedingt wieder in seine Ruheposition zurück.

In Fig. 8a ist eine Schnittansicht des Deckels 2 mit montiertem Griff 1 entlang der Linie VIIIa der Fig. 6 zu sehen.

In Fig. 8b ist eine vergrößerte Darstellung des Bereichs Vlllb der Fig. 8a gezeigt, die den Griff 1 in derselben ausgelenkten Stellung wie in Fig. 7b zeigt, folglich in genau der Position, in der die Außenkante 26 des Griffstegs 14 mit dem Deckel 2 in Anlage tritt. In dieser Position kann der Griff 1 in einer Richtung (im Wesentlichen) senkrecht zur Deckeloberseite nicht weiter von der Deckeloberseite weg bewegt werden. Dies wird beispielsweise dadurch realisiert, dass in der gezeigten Relativlage von Fixierungslasche 10 und Befestigungsstrebe 22 der Innendurchmesser der Fixierungslasche 10 unterhalb der Befestigungsstrebe 22 gleich oder kleiner als die Breite der Befestigungsstrebe 22 ist. Unter der Breite der Befestigungsstrebe 22 wird ihre Ausdehnung entlang der Deckeloberseite in einer Richtung von den Fixierungslaschen 10 hin zur Ausnehmung 24 verstanden. Unterhalb der Befestigungsstrebe 22 bedeutet in diesem Zusammenhang eine von der Deckeloberseite abgekehrte Richtung.

Fig. 9 zeigt eine perspektivische Draufsicht des Deckels 2 in der Ruheposition des Griffs 1, in der der Griff 1 (im Wesentlichen) lückenlos auf dem Deckel 2 aufliegt und die Fixierungslaschen 10 sowie die Greiflasche 20 in den Deckel 2 hinein versenkt sind. Dies ist insbesondere mit Blick auf die Fig. 10 zu erkennen.

In Fig. 10 ist eine Seitenansicht des in Fig. 9 gezeigten Deckels 2 in der erfindungsgemäßen Ausführungsform in einer Ruheposition des Griffs 1 gezeigt. Zu sehen ist, dass der Griff 1 bzw. der Grundkörper 4 des Griffs 1 mit seiner Unterseite 4b (im Wesentlichen) lückenlos auf dem Deckel 2 aufliegt (siehe auch Fig. 11) und die Gesamthöhe des Griffs 1, die dieser auf den Deckel 2 aufbaut, nicht mehr beträgt als die Stärke des Metallblechs, aus dem der Griff 1 gefertigt ist. In dieser Position sind die Fixierungslaschen 10 und die Greiflasche 20 in den Deckel 2 hinein versenkt bzw. greifen durch diesen hindurch.

Fig. 11 zeigt eine Schnittansicht des Griffs 1 in der erfindungsgemäßen Ausführungsform im Bereich einer Fixierungslasche 10 in der Ruheposition des Griffs 1. In der Ruheposition liegt der Grundkörper 4 mit der Unterseite 4b am Deckel 2 an und die Fixierungslasche 10 umschließt die Befestigungsstrebe 22. Dabei liegt der Grundkörper 4 auch auf der Befestigungsstrebe 22, die einen Teil der Deckeloberseite bildet, an.

In Fig. 12 ist eine Draufsicht auf ein zugeschnittenes Metallblech bzw. einen Blechrohling, aus dem der Griff 1 gefertigt wird, gezeigt. Der Griff 1 wird durch Umformen des gezeigten Blechrohlings gefertigt. Der Blechrohling weist den Grundkörper 4 mit dem Griffsteg 6, den beiden Halteschenkeln 12 und dem Aussteifungssteg 14 sowie zwei laschenartige Vorsprünge auf, die jeweils an dem Ende der Halteschenkel 12 angeordnet sind, das dem Griffsteg 6 abgewandt ist. auf. An dem Griffsteg 6 ist an einem Innenkantenabschnitt 18 ein Blechband ausgeformt, das den Griffsteg 6 in Richtung hin zu den laschenartigen Vorsprüngen verbreitert. Die Form des gezeigten Blechrohlings kann durch (Aus)Stanzen eines gewöhnlichen Metallblechs gebildet sein.

Um den Griff 1 aus dem gezeigten Blechrohling zu formen, werden die laschenartigen Vorsprünge durch Umformen, beispielsweise Biegen, in die in Fig. 4 gezeigte offen gekrümmte Form gebracht und bilden jeweils eine Fixierungslasche 10 Das Blechband wird ebenfalls durch Umformen, beispielsweise Biegen, in die in Fig. 3 gezeigte Form gebracht und bildet die Greiflasche 20. Das Metallblech hat nun die Form des Halbfertigartikels des Griffs 1, in der der Griff 1 an den Deckel 2 montiert werden kann.

Zur Montage des halbfertigen Griffs 1 mit dem Deckel 2 werden die Fixierungslaschen 10 um die Befestigungsstreben 22 des Deckels 2 geführt bzw. werden die Befestigungsstreben 22 durch den Spalt zwischen den freien Enden 16 und der Unterseite 4b hindurch geführt. Danach werden die Fixierungslaschen 10 weiter in Richtung der Unterseite 4b gedrückt und umgeformt, sodass die freien Enden 16 auf der Unterseite 4b zum Liegen kommen und die Fixierungslaschen 10 geschlossen sind. Nun ist der Griff 1 am Deckel 2 schwenkbar gesichert.

Es wird verständnishalber angemerkt, dass der gesamte Griff einschließlich Grundkörper und aller Laschen einstückig aus einem Blech gefertigt ist.

### Bezugszeichenliste

- 1: Griff
- 2: Deckel
- 4: Grundkörper
- 4a: Oberseite des Grundkörpers
- 4b: Unterseite des Grundkörpers
- 6: Griffsteg
- 8: Halteabschnitt
- 10: Fixierungslasche
- 10a1-10a3: Gekrümmte Abschnitte der Fixierungslasche
- 10b1-10b3: Lineare Abschnitte der Fixierungslasche
- 12: Halteschenkel
- 14: Aussteifungssteg
- 16: freies Ende der Fixierungslasche
- 18: Innenkante des Griffstegs
- 20: Greiflasche
- 22: Befestigungsstrebe
- 24: Ausnehmung
- 26: Außenkante des Aussteifungsstegs
- 28: Gitterstrebe

## Patentansprüche

1. Griff (1) für einen Deckel (2) eines medizinischen Siebkorbs, mit
einem Grundkörper (4), der einen Griffsteg (6) und einen sich an den Griffsteg (6) anschließenden Halteabschnitt (8) aufweist oder ausbildet, und
mindestens einer Ösen-förmigen oder gebogenen Fixierungslasche (10), die an einem dem Griffsteg (6) entgegengesetzten Ende des Halteabschnitts (8) zur Schwenkmontage des Griffs (1) angeordnet ist, **dadurch gekennzeichnet, dass**
der Grundkörper (4) einschließlich des Griffstegs (6) und des Halteabschnitts (8) aus einem planen Metallblech besteht, welches an dem dem Griffsteg (6) entgegengesetzten Ende des Halteabschnitts (8) zu zumindest einem, diesen in einer dem Griffsteg (6) entgegengesetzten Richtung verlängernden, laschenartigen Vorsprung stoffeinstückig ausgearbeitet ist, welcher zur Ausbildung der zumindest einen Fixierungslasche (10) in Bezug auf den Halteabschnitt (8), vorzugsweise zu einer Öse, umgebogen ist, so dass die zumindest eine umgebogene Fixierungslasche (10) über ihre Breite zur Schwenkmontage des Griffs (1) ausgebildet ist.

2. Griff (1) nach Anspruch 1, wobei der Halteabschnitt (8) zwei Halteschenkel (12) aufweist oder aus diesen besteht, an deren freien Enden/Endabschnitten jeweils eine Fixierungslasche (10) angeordnet oder ausgebildet ist, und sich insbesondere an den freien Enden des Griffstegs (6) jeweils ein Halteschenkel (12) vorzugsweise im Wesentlichen senkrecht an diesen anschließt, wobei die Griffsteglänge zwischen den Halteschenkeln (12) wie auch die Halteschenkellängen bevorzugt für ein vollumfänglichen Umgreifen des Griffstegs durch eine Menschenhand in jeder Schwenkposition des Griffs (1) dimensioniert sind.

3. Griff (1) nach Anspruch 2, wobei der Halteabschnitt (8) weiter einen Aussteifungssteg (14) aufweist, der die zwei Halteschenkel (12) zwischen Griffsteg (6) und Fixierungslasche (10), insbesondere im Bereich vorzugsweise nahe der freien Enden der Halteschenkel (12), stoffeinstückig verbindet.

4. Griff (1) nach Anspruch 3, wobei die Position des Aussteifungsstegs (14) relativ zu der mindestens einen Fixierungslasche (10) und/oder die Form des Aussteifungsstegs (14) derart bestimmt ist, dass der Aussteifungssteg (14) zusätzlich einen Schwenkanschlag des Griffs (1) bildet, der dafür vorgesehen und ausgebildet ist, mit Erreichen einer ausgeschwenkten Lage des Griffs (1) bevorzugt mit dem Deckel (2) oder einem Schwenkscharnier des Griffs (1) in Schwenkstopp-Anlage zu kommen.

5. Griff (1) nach einem der vorstehenden Ansprüche 2 bis 4, wobei die aus einem planen Metallblech umgebogene Fixierungslasche (10) folgende Längsabschnitte in der angegebenen Reihenfolge ausgehend vom jeweiligen Halteschenkel (12) hat:
- ein erster Krümmungsabschnitt (10a1), der die Fixierungslasche (10) ausgehend vom Halteschenkel (12) um einen ersten stumpfen Winkel vorzugsweise zwischen 90° und 110° zum Halteschenkel (12) abkrümmt,
- einen ersten geraden Abschnitt (10b1) von einer ersten Länge, der sich an den ersten Krümmungsabschnitt (10a1) anschließt,
- einen zweiten Krümmungsabschnitt (10a2), der den ersten geraden Abschnitt (10b1) um einen zweiten stumpfen Winkel in die gleiche Abkrümmrichtung wie der erste Krümmungsabschnitt (10a1) abkrümmt, wobei der zweite stumpfe Winkel größer ist als der erste stumpfe Winkel, vorzugsweise zwischen 120 und 150°,
- einen zweiten geraden Abschnitt (10b2), der sich an den zweiten Krümmungsabschnitt (10a2) anschließt und eine zweite Länge hat, die größer ist als die erste Länge,
- einen dritten Krümmungsabschnitt (10a3), der den zweiten geraden Abschnitt (10b2) um einen dritten stumpfen Winkel in die gleiche Abkrümmrichtung wie der zweite Krümmungsabschnitt (10a2) abkrümmt, wobei der dritte stumpfe Winkel (10b1) im Wesentlichen dem ersten stumpfen Winkel entspricht, der Krümmungsradius des dritten Krümmungsabschnitts (10a3) jedoch größer ist als der Krümmungsradius des ersten Krümmungsabschnitts (10a1) und
- einen dritten geraden Abschnitt (10b3), der sich an den dritten Krümmungsabschnitt (10a3) anschließt und eine dritte Länge hat, die im Wesentlichen der ersten Länge entspricht.

6. Griff (1) nach einem der vorstehenden Ansprüche 1 bis 5, wobei an einem Innenkantenabschnitt (18) des Griffstegs (6) zumindest eine rinnen- oder muldenförmige Greiflasche (20) ausgebildet ist.

7. Griff (1) nach Anspruch 6, wobei zur Ausbildung der Greiflasche (20) an dem Innenkantenabschnitt (18) ein Blechband, das den Griffsteg (6) in Richtung hin zum zumindest einen laschenartigen Vorsprung verbreitert, ausgeformt und umgebogen ist.

8. Deckel (2) für einen medizinischen Siebkorb, mit einem Griff (1) nach einem der Ansprüche 1 bis 5 und mit zumindest einer Befestigungsstrebe (22) oder zumindest einem Scharnierstift, die/der von der mindestens einen Fixierungslasche (10) umfangsseitig umgriffen ist und an die/den der Griff (1) schwenkbar befestigt ist.

9. Deckel (2) nach Anspruch 8, wobei in einer Ruheposition des Griffs (1), in der dieser mit seinem Grundkörper (4) am Deckel (2) anliegt, sich die mindestens eine Fixierungslasche (10) auf der dem Deckel (2) zugewandten Griffseite befindet.

10. Deckel (2) nach Anspruch 9, wobei der Deckel (2) eine Ausnehmung (24) oder Mulde aufweist, die so positioniert und dazu vorgesehen und angepasst ist, dass die Greiflasche (20) in der Ruheposition des Griffs (1) in die Ausnehmung (24) oder Mulde hinein versenkt ist.

11. Deckel (2) nach einem der Ansprüche 8 bis 10, wobei die Deckelseite, die in der Ruheposition des Griffs (1) dem Griff (1) zugewandt ist, eine Deckeloberseite ist, und die Befestigungsstrebe (22) nicht über die Deckeloberseite hinausragt, insbesondere bündig mit der Deckeloberseite ist.

12. Deckel (2) nach einem der Ansprüche 8 bis 11, wobei eine Auslenkung des Griffs (1) relativ zum Deckel (2) auf einen maximalen Auslenkungswinkel von 80° bis 89°, insbesondere 85° begrenzt ist.

13. Deckel (2) nach Anspruch 12, wobei die Relativlage zwischen der dem Deckel (2) zugewandten Unterkante des Halteabschnitts (8), insbesondere des Aussteifungsstegs (14), und der mindestens einen Fixierungslasche (10) derart bestimmt ist, dass mit Erreichen des maximalen Auslenkungswinkels die Unterkante auf der Deckeloberseite anschlägt.

14. Verfahren zum Bilden eines Griffs (1) nach einem der Ansprüche 1 bis 5 an einem Deckel (2) nach einem der Ansprüche 8 bis 11 aus einem Blechrohling, der einen Grundkörper (4) bestehend aus einem Griffsteg (6) und zumindest einem sich an diesen anschließenden Halteabschnitt (8) und zumindest einen, den Halteabschnitt (8) verlängernden, laschenartigen Vorsprung mit einem vom Halteabschnitt (8) abgewandten freien Ende (16) aufweist, mit den Schritten:
Umformen des zumindest einen laschenartigen Vorsprungs zu einer umgebogenen Fixierungslasche (10) derart, sodass diese eine offene, gekrümmte Form einnimmt;
Einbringen einer Befestigungsstrebe (22) des Deckels (2) in die offene, gekrümmte Fixierungslasche (10), sodass diese die Befestigungsstrebe (22) offen umgreift; und
Umformen der offenen Fixierungslasche (10) derart, dass ihr freies Ende (16) näher an den Grundkörper (4) angeordnet wird und/oder das freie Ende (16) am Grundkörper (4) zum Liegen kommt und die Befestigungsstrebe (22) von der Fixierungslasche (10) umschlossen und schwenkbar gesichert wird.

15. Verfahren nach Anspruch 14, **dadurch gekennzeichnet, dass** der Blechrohling weiter an einer Innenkante (18) des Griffstegs (6) zu zumindest einem, diesen in Richtung hin zum laschenartigen Vorsprung verbreiternden Blechband ausgeformt ist und das Verfahren weiter den folgenden Schritt aufweist:
Umformen des Blechbands derart, dass dieses eine umgebogene Greiflasche (20) bildet.

## Claims

1. Handle (1) for a cover (2) of a medical sieve basket, comprising
a base body (4) comprising or forming a handle bar (6) and a holding portion (8) adjoining the handle bar (6), and
at least one eyelet-shaped or curved fixing lug (10) arranged at an end of the holding portion (8) opposite the handle bar (6) for pivot mounting of the handle (1), **characterized in that**
the base body (4) including the handle bar (6) and the holding portion (8) consists of a planar metal sheet which, at the end of the holding portion (8) opposite the handle bar (6), is formed as a single piece of material into at least one lug-like projection extending it in a direction opposite the handle bar (6), said projection being bent to form the at least one fixing lug (10) with respect to the holding portion (8), preferably to form an eyelet, so that the at least one bent fixing lug (10) is formed over its width for pivot mounting of the handle (1).

2. Handle (1) according to claim 1, wherein the holding portion (8) has or consists of two holding legs (12), at the free ends/end portions of which a respective fixing lug (10) is arranged or formed, and a respective holding leg (12) adjoins the handle bar (6), preferably substantially perpendicularly, in particular at the free ends thereof, wherein the handle bar length between the holding legs (12) as well as the holding leg lengths are preferably dimensioned for completely encompassing the handle bar around its circumference by a human hand in any pivot position of the handle (1).

3. Handle (1) according to claim 2, wherein the holding portion (8) further comprises a stiffening bar (14) that integrally connects the two holding legs (12) between handle bar (6) and fixing lug (10), in particular in the region preferably near the free ends of the holding legs (12).

4. Handle (1) according to claim 3, wherein the position of the stiffening bar (14) relative to the at least one fixing lug (10) and/or the shape of the stiffening bar (14) is determined in such a way that the stiffening bar (14) additionally forms a pivot stop of the handle (1), which is provided and designed for coming into pivot stop contact preferably with the cover (2) or a pivot hinge of the handle (1) upon reaching a swungout position of the handle (1).

5. Handle (1) according to one of the preceding claims 2 to 4, wherein the fixing lug (10) bent from a planar metal sheet has the following longitudinal portions in the indicated order starting from the respective holding leg (12):
- a first bending portion (10a1) which bends the fixing lug (10) starting from the holding leg (12) by a first obtuse angle preferably between 90° and 110° with respect to the holding leg (12),
- a first straight portion (10b1) of a first length joining the first bending portion (10a1),
- a second bending portion (10a2) bending the first straight portion (10b1) by a second obtuse angle in the same bending direction as the first bending portion (10a1), wherein the second obtuse angle is larger than the first obtuse angle, preferably between 120 and 150°,
- a second straight portion (10b2) joining said second bending portion (10a2) and having a second length greater than said first length,
- a third bending portion (10a3) that bends the second straight portion (10b2) through a third obtuse angle in the same bending direction as the second bending portion (10a2), wherein the third obtuse angle (10b1) substantially corresponds to the first obtuse angle, but the bending radius of the third bending portion (10a3) is greater than the bending radius of the first bending portion (10a1), and
- a third straight portion (10b3) joining the third bending portion (10a3) and having a third length substantially equal to the first length.

6. Handle (1) according to one of the preceding claims 1 to 5, wherein at least one groove-shaped or trough-shaped gripping lug (20) is formed on an inner edge portion (18) of the handle bar (6).

7. Handle (1) according to claim 6, wherein a sheet metal band that widens the handle bar (6) toward the at least one lug-like projection is formed and bent to form the gripping lug (20) at the inner edge portion (18).

8. Cover (2) for a medical sieve basket, having a handle (1) according to one of claims 1 to 5 and having at least one fixing strut (22) or at least one hinge pin, which is embraced circumferentially by the at least one fixing lug (10) and to which the handle (1) is pivotably fastened.

9. Cover (2) according to claim 8, wherein in a rest position of the handle (1), in which it rests with its base body (4) against the cover (2), the at least one fixing lug (10) is located on the handle side facing the cover (2).

10. Cover (2) according to claim 9, wherein the cover (2) includes a recess (24) or trough positioned and provided for and adapted such that the gripping lug (20) is countersunk into the recess (24) or trough in the rest position of the handle (1).

11. Cover (2) according to one of claims 8 to 10, wherein the cover side facing the handle (1) in the rest position of the handle (1) is an upper side of the cover, and the fixing strut (22) does not project beyond the upper side of the cover, in particular is flush with the upper side of the cover.

12. Cover (2) according to one of claims 8 to 11, wherein a deflection of the handle (1) relative to the cover (2) is limited to a maximum deflection angle of 80° to 89°, in particular 85°.

13. Cover (2) according to claim 12, wherein the relative position between the lower edge of the holding portion (8) facing the cover (2), in particular of the stiffening bar (14), and the at least one fixing lug (10) is determined in such a way that, when the maximum deflection angle is reached, the lower edge abuts on the upper side of the cover.

14. Method for forming a handle (1) according to one of claims 1 to 5 on a cover (2) according to one of claims 8 to 11 from a sheet metal blank, which has a base body (4) consisting of a handle bar (6) and at least one holding portion (8) adjoining the latter and at least one lug-like projection extending the holding portion (8) and having a free end (16) facing away from the holding portion (8), having the steps:
reshaping the at least one lug-like projection into a bent fixing lug (10) such that it assumes an open, curved shape;
inserting a fixing strut (22) of the cover (2) into the open, curved fixing lug (10) so that it openly embraces the fixing strut (22); and
reshaping the open fixing lug (10) such that its free end (16) is positioned closer to the base body (4) and/or the free end (16) comes to rest on the base body (4) and the fixing strut (22) is enclosed and pivotally secured by the fixing lug (10).

15. Method according to claim 14, **characterized in that** the sheet metal blank is further formed at an inner edge (18) of the handle bar (6) into at least one sheet metal band widening the same towards the lug-like projection, and the method further comprises the step of:
reshaping the sheet metal band such that it forms a bent gripping lug (20).

## Revendications

1. Poignée (1) pour un couvercle (2) d'un panier de tamisage médical, avec un corps de base (4) qui présente ou forme une traverse de poignée (6) et une section de maintien (8) qui se raccorde à la traverse de poignée (6), et
au moins une bride de fixation (10) en forme d'œillet ou courbée qui est agencée au niveau d'une extrémité de la section de maintien (8), laquelle extrémité est opposée à la traverse de poignée (6), pour un montage en pivotement de la poignée (1), **caractérisée en ce que**
le corps de base (4), y compris la traverse de poignée (6) et la section de maintien (8), est constitué d'une tôle métallique plane, laquelle est travaillée en une seule pièce matérielle au niveau de l'extrémité de la section de maintien (8), laquelle extrémité est opposée à la traverse de poignée (6), pour former au moins un épaulement en forme de bride et qui rallonge ladite section de maintien dans une direction opposée à la traverse de poignée (6), lequel est recourbé, de préférence en forme d'œillet, pour former la au moins une bride de fixation (10) par rapport à la section de maintien (8), de sorte que la au moins une bride de fixation (10) recourbée soit conçue sur sa largeur pour un montage en pivotement de la poignée (1).

2. Poignée (1) selon la revendication 1, dans laquelle la section de maintien (8) présente deux ailes de maintien (12) ou en est constituée, au niveau des extrémités libres/sections d'extrémité desquelles est respectivement agencée ou conçue une bride de fixation (10), et une aile de maintien (12) se raccorde respectivement, en particulier au niveau des extrémités libres de la traverse de poignée (6), de préférence essentiellement à la perpendiculaire de celles-ci, dans laquelle la longueur de traverse de poignée entre les ailes de maintien (12) ainsi que les longueurs d'aile de maintien sont dimensionnées de préférence pour entourer entièrement la traverse de poignée par une main d'homme dans chaque position de pivotement de la poignée (1).

3. Poignée (1) selon la revendication 2, dans laquelle la section de maintien (8) présente en outre une traverse de renforcement (14) qui relie en une seule pièce matérielle les deux ailes de maintien (12) entre la traverse de poignée (6) et la bride de fixation (10), en particulier dans la région de préférence près des extrémités libres des ailes de maintien (12).

4. Poignée (1) selon la revendication 3, dans laquelle la position de la traverse de renforcement (14) par rapport à la au moins une bride de fixation (10) et/ou la forme de la traverse de renforcement (14) est déterminée de telle sorte que la traverse de renforcement (14) forme en outre une butée de pivotement de la poignée (1), laquelle butée de pivotement est prévue et conçue pour aller dans une installation d'arrêt de pivotement dès lors qu'une situation pivotée vers l'extérieur de la poignée (1) a été atteinte, de préférence avec le couvercle (2) ou avec une charnière pivotante de la poignée (1).

5. Poignée (1) selon l'une des revendications précédentes 2 à 4, dans laquelle la bride de fixation (10) recourbée fabriquée dans une tôle métallique plane présente les sections longitudinales suivantes dans l'ordre indiqué en partant de l'aile de maintien (12) respective :
- une première section de courbure (10a1) qui incurve la bride de fixation (10) en partant de l'aile de maintien (12) selon un premier angle obtus de préférence entre 90° et 110° par rapport à l'aile de maintien (12),
- une première section rectiligne (10b1) d'une première longueur, qui se raccorde à la première section de courbure (10a1),
- une deuxième section de courbure (10a2) qui incurve la première section rectiligne (10b1) selon un deuxième angle obtus dans la même direction de courbure que la première section de courbure (10a1), dans laquelle le deuxième angle obtus est supérieur au premier angle obtus, de préférence entre 120° et 150°,
- une deuxième section rectiligne (10b2) qui se raccorde à la deuxième section de courbure (10a2) et présente une deuxième longueur qui est supérieure à la première longueur,
- une troisième section de courbure (10a3) qui incurve la deuxième section rectiligne (10b2) selon un troisième angle obtus dans la même direction de courbure que la deuxième section de courbure (10a2), dans laquelle le troisième angle obtus (10b1) correspond essentiellement au premier angle obtus, le rayon de courbure de la troisième section de courbure (10a3) étant cependant supérieur au rayon de courbure de la première section de courbure (10a1) et
- une troisième section rectiligne (10b3) qui se raccorde à la troisième section de courbure (10a3) et présente une troisième longueur qui correspond essentiellement à la première longueur.

6. Poignée (1) selon l'une des revendications précédentes 1 à 5, dans laquelle au moins une bride de préhension (20) en forme de goulotte ou d'auge est conçue au niveau d'une section de bord intérieur (18) de la traverse de poignée (6).

7. Poignée (1) selon la revendication 6, dans laquelle une bande de tôle qui élargit la traverse de poignée (6) dans la direction qui va vers le au moins un épaulement en forme de bride est façonnée et incurvée pour former la bride de préhension (20) au niveau de la section de bord intérieur (18).

8. Couvercle (2) pour un panier de tamisage médical, avec une poignée (1) selon l'une des revendications 1 à 5 et avec au moins une barre de fixation (22) ou au moins une goupille de charnière, qui est entourée sur le pourtour par la au moins une bride de fixation (10) et au niveau de laquelle la poignée (1) est fixée en pivotement.

9. Couvercle (2) selon la revendication 8, dans lequel, dans une position de repos de la poignée (1) où celle-ci est appliquée contre le couvercle (2) avec son corps de base (4), la au moins une bride de fixation (10) se trouve sur le côté de poignée tourné vers le couvercle (2).

10. Couvercle (2) selon la revendication 9, dans lequel le couvercle (2) présente un évidement (24) ou une auge qui est positionné(e) de telle sorte que et prévu(e) et adapté(e) pour que la bride de préhension (20) soit enfoncée jusque dans l'évidement (24) ou l'auge dans la position de repos de la poignée (1).

11. Couvercle (2) selon l'une des revendications 8 à 10, dans lequel le côté de couvercle qui est tourné vers la poignée (1) dans la position de repos de la poignée (1) est un côté supérieur de couvercle, et la barre de fixation (22) ne fait pas saillie par-dessus le côté supérieur de couvercle, en particulier n'affleure pas sur le côté supérieur de couvercle.

12. Couvercle (2) selon l'une des revendications 8 à 11, dans lequel une déviation de la poignée (1) par rapport au couvercle (2) est limitée à un angle maximal de déviation de 80° à 89°, en particulier 85°.

13. Couvercle (2) selon la revendication 12, dans lequel la situation relative entre le bord inférieur de la section de maintien (8) tourné vers le couvercle (2), en particulier de la traverse de renforcement (14), et la au moins une bride de fixation (10) est déterminée de telle sorte que dès lors que l'angle maximal de déviation a été atteint, le bord inférieur vient buter contre le côté supérieur de couvercle.

14. Procédé de formation d'une poignée (1) selon l'une des revendications 1 à 5 au niveau d'un couvercle (2) selon l'une des revendications 8 à 11 à partir d'une ébauche en tôle, qui présente un corps de base (4), constitué d'une traverse de poignée (6) et d'au moins une section de maintien (8) qui se raccorde à celle-ci, et au moins un épaulement en forme de bride et qui rallonge la section de maintien (8) avec une extrémité libre (16) opposée à la section de maintien (8), avec les étapes consistant à :
façonner le au moins un épaulement en forme de bride en une bride de fixation (10) recourbée, de sorte que celle-ci prenne une forme ouverte et courbée ;
introduire une barre de fixation (22) du couvercle (2) dans la bride de fixation (10) ouverte et courbée, de sorte que la barre de fixation (22) entoure de façon ouverte ; et
façonner la bride de fixation (10) ouverte de sorte que son extrémité libre (16) soit agencée plus près du corps de base (4) et/ou que l'extrémité libre (16) en arrive à reposer contre le corps de base (4) et que la barre de fixation (22) soit enserrée et sécurisée de manière pivotante par la bride de fixation (10).

15. Procédé selon la revendication 14, **caractérisé en ce que** l'ébauche en tôle est en outre façonnée, au niveau d'un bord intérieur (18) de la traverse de poignée (6), en au moins une bande de tôle qui élargit l'épaulement en forme de bride dans la direction de ladite traverse de poignée, et le procédé présente en outre l'étape suivante consistant à :
façonner la bande de tôle de sorte que celle-ci forme une bride de préhension (20) courbée.
